# EUROPÄISCHE PATENTSCHRIFT

(11) **EP 3 655 068 B1**
(45) Veröffentlichungstag und Bekanntmachung des Hinweises auf die Patenterteilung: **25.12.2024**
(21) Anmeldenummer: 18740167.4
(22) Anmeldetag: 06.07.2018
(51) Int. Cl.: A61M 5/168, E05C 1/00, E05C 1/06, E05C 9/02, E05B 17/00

(54) **VORRICHTUNG ZUM ÖFFNEN UND SCHLIESSEN EINER SCHLAUCHKLEMME INNERHALB EINES PUMPENGEHÄUSES**
DEVICE FOR OPENING AND CLOSING OF A TUBE CLAMP WITHIN A PUMP HOUSING
DISPOSITF POUR OUVRIR ET FERMER UNE PINCE POUR TUYAUX SOUPLES DANS CORPS DE POMPE

(30) Priorität: 18.07.2017 DE 102017116105
(43) Veröffentlichungstag der Anmeldung: 27.05.2020
(73) Patentinhaber: B. Braun Melsungen AG, 34212 Melsungen (DE)
(72) Erfinder: PRZYKLENK, Arthur, 34131 Kassel (DE)
(74) Vertreter: Winter, Brandl - Partnerschaft mbB
(86) Internationale Anmeldenummer: PCT/EP2018/068383
(87) Internationale Veröffentlichungsnummer: WO 2019/015999

(56) Entgegenhaltungen:
- DE-B- 1 166 040
- US-A- 3 341 239
- US-A- 4 025 096
- US-A- 4 254 976

## Beschreibung

Die Erfindung betrifft eine Vorrichtung zum Öffnen und Schließen insbesondere einer innerhalb eines Pumpengehäuses angeordneten Schlauchklemme zur sicheren Endlagensteuerung eines Griffs.

### Hintergrund der Erfindung

Beispielsweise in der Medizintechnik sind Pumpen wie z.B. Infusionsschlauchpumpen bekannt, bei denen das zu fördernde Medium durch äußere mechanische Verformung eines Schlauches mittels eines Rotorkopfes durch diesen hindurchgedrückt wird (Quetschpumpenprinzip). Bei derartigen Pumpen werden standardmäßig Sicherheits- oder Schlauchklemmen an dem Schlauch eingesetzt, um einen sogenannten "Free-Flow" zu verhindern, kurz FFC (free-flow clamp). Unter der Bezeichnung "Free-Flow" ist dabei der Gravitationsfluss zu verstehen, der eventuell in einer gefährlichen Überinfusion resultieren kann. Die Klemmen befinden sich standardmäßig direkt am (Infusions-)Schlauch, um einen Durchfluss eines Infusionsfluides durch den Infusionsschlauch zu unterbinden oder zu ermöglichen. Zum Einlegen eines (Infusions-)Schlauches in eine (Infusions-)Pumpe wird meist eine an einem Pumpengehäuse anscharnierte Pumpenklappe geöffnet und bei stationären Sicherheitsklemmen in der Pumpe der Schlauch in diese eingelegt.

Im Allgemeinen werden Sicherheitsklemmen meist indirekt durch/über eine manuelle Betätigung der Pumpenklappe der Pumpe zwangsmäßig geöffnet oder geschlossen, um quasi automatisch ein ungewolltes Durchfließen des Fluids durch den Schlauch zu unterbinden. Hierbei kann es auch vorkommen, dass die Sicherheitsklemme direkt manuell geschlossen werden muss, bevor die Pumpenklappe ordnungsgemäß geschlossen werden kann. Oftmals verhält es sich aber so, dass die schlauchseitige Sicherheitsklemme zuerst direkt manuell geschlossen wird und dann aber mit dem Schließen der Pumpenklappe wieder indirekt (automatisch) geöffnet wird. Eine nach dem Schließen der Pumpenklappe geschlossene Schlauchklemme kann mittels eines Aktuators der Pumpe für den Pumpenbetrieb automatisch wieder direkt geöffnet werden. Neben Pumpen mit einer derartigen Öffnungsfunktion existieren Aktuatoren für eine entsprechende Schließfunktion zum Schließen der schlauchseitigen Sicherheitsklemme.

Bei bekannten Vorrichtungen zum Öffnen und Schließen beginnt die Vorrichtung bei manueller Betätigung eines Griffs/Griffhebels mit dem Öffnungs- oder Schließvorgang, indem ein Rückhalteelement direkt oder indirekt freigegeben wird. Der Griffhebel ist in der Regel scharnierartig an einem Gehäuse oder an einer Klappe gelagert, welche zum Verschließen einer Gehäuseöffnung vorgesehen ist, wobei die Schwenkbewegung in eine Axialbewegung eines Schließriegels übertragen wird, der so mit dem Rückhalteelement in und außer Eingriff bringbar ist.

### Stand der Technik

Die DE 4310878 offenbart beispielsweise einen Griff für Fenster, Türen oder dergleichen. In diesem Fall bildet ein Lenker mit einer Kurbel ein Kniehebel-Paar. Ein Handgriff dreht sich um eine Kurbelwelle und bildet somit den Hebel zu der Kurbel. Diese wiederum ist schwenkbar mittels eines Kniehebels-Drehlagers an einem Lenker befestigt. Der Lenker wiederum ist schwenkbar an einer Schiebeplatte befestigt. Die Aufgabe dieser Anordnung ist es eine kleine Dimensionierung zu erreichen. Grundsätzlich besteht bei Griffhebelkonstruktionen zur Betätigung/Axialverschiebung eines Schließriegels das Problem, dass durch Rückstellkräfte aber auch durch erhöhte Reibung im Betätigungskraft-Übertragungszug und/oder zwischen Schließriegel und Riegelführung es in Verbindung mit Unachtsamkeiten seitens des Benutzers dazu kommen kann, dass der Griff/Griffhebel sich nach seiner manuellen Betätigung nicht vollständig in einer Schließposition befindet, wobei die Schließposition jene Position ist, bei der sich der Griff in seiner Endposition für den Schließvorgang befindet. Ein unbeabsichtigtes Öffnen des Schließmechanismus vor allem im medizinischen Bereich kann dann zu Gefährdungen führen.

Sollte dem Schließmechanismus zudem weitere Funktionen zugeordnet sein wie das vorstehend beschriebene Betätigen beispielsweise einer Sicherheits-/Schlauchklemme parallel zur Schließbewegung des Schließriegels, besteht die zusätzliche Gefahr, dass bei nicht Erreichen der Schließposition des Griffhebels auch die Sicherheits-/Schlauchklemme nicht vollständig in deren Klemmposition verbracht wird, in der sie normaler Weise durch einen integrierten Rastmechanismus verrastet. Es ist daher von herausragender Bedeutung, dass der Griffhebel einer Vorrichtung zum Öffnen und Schließen insbesondere im medizinischen Bereich sicher seine Endposition gemäß vorstehender Definition erreicht und diese hält.

Des Weiteren ist aus der DE 11 66 040 eine Öffnungs- und Schließvorrichtung bekannt, mit einem Betätigungshebel, der an einem Schwenkbolzen eines Zwischenglieds angelenkt ist, über den ein Verriegelungsstab betätigt werden kann.

Die US 3 341 239 offenbart ferner einen Griff an einer Tür, mittels welchem über ein daran anscharniertes Bein und einer am Bein anscharnierten Armwippe ein (Verriegelungs-) Pin bewegt werden kann. In anderen Worten ausgedrückt besteht das Hebelgelenk gemäß der US 3 341 239 somit aus zwei Bauteilen, nämlich dem Bein und der Armwippe, zur Kraftübertragung vom Griff auf den (Verriegelungs-) Pin.

Die US 4 025 096 offenbart schließlich einen Tür-Öffnungs- und Schließmechanismus gemäß dem Oberbegriff des Patentanspruchs 1.

### Kurzbeschreibung der Erfindung

Demzufolge liegt der vorliegenden Erfindung die Aufgabe zugrunde, eine Vorrichtung zum Öffnen und Schließen (Schließmechanismus) bereitzustellen, welche ein erschütterungssicheres und positionssicheres Verschließen zumindest einer Klappe/Tür oder dergleichen ermöglicht, so dass ein unbeabsichtigtes Öffnen erschwert wird. Zugleich soll die Vorrichtung zum Öffnen und Schließen aber auch kompatibel und günstig in der Herstellung und des Weiteren mit wenig Kraftaufwand zu öffnen sein.

Diese Aufgabe wird durch eine Vorrichtung zum Öffnen und Schließen einer Tür oder Klappe mit den Merkmalen des Anspruchs 1 gelöst. Weitere vorteilhafte Ausführungsformen sind in den Unteransprüchen beansprucht.

Das Grundkonzept der vorliegenden Erfindung sieht darüber hinaus eine Vorrichtung zum Öffnen und Schließen, nachfolgend auch einfach als Schließmechanismus bezeichnet, vor, mit einem Griffhebel, der in seinem Mittenabschnitt an ein Gehäuse, Klappe oder Tür wippenartig anscharnierbar oder anscharniert ist, wodurch der Griffhebel in dessen Längsrichtung in einen (langen) Betätigungsabschnitt und einen (kurzen) Aktionsabschnitt unterteilt ist, an dessen Endbereich ein Hebelgelenk anscharniert ist, das wiederum an eine Schieberkulisse oder Riegel angelenkt ist. Erfindungsgemäß erfüllen die drei vorstehend genannten Anlenkpunkte/Anlenkpositionen, nämlich zwischen Griffhebel und Gehäuse/ Klappe/ Tür (nachfolgend als "erstes Scharnier" bezeichnet) sowie zwischen Griffhebel und Hebelgelenk (nachfolgend als "zweites Scharnier" bezeichnet) sowie zwischen Hebelgelenk und Schieberkulisse (nachfolgend als "drittes Scharnier" bezeichnet) die folgende Raumgeometrie:
- In einer bestimmten Betätigungszwischenposition vor Erreichen der vorstehend definierten Endposition des Griffhebels befinden sich das erste bis dritte Scharnier auf einer Linie.
- Mit Erreich der vorstehend definierten Endposition des Griffhebels befinden sich das erste und dritte Scharnier auf einer Linie, wohingegen das zweite Scharnier (senkrecht) zu dieser Linie beabstandet ist und zwar in Öffnungsrichtung des Griffhebels.
- Spätestens mit Erreichen der (vollständigen) Öffnungsposition des Griffhebels insbesondere mit Überschreiten der bestimmten Betätigungszwischenposition in Richtung Öffnungsposition liegen das erste und dritte Scharnier auf einer Linie, wohingegen das zweite Scharnier (senkrecht) zu dieser Linie beabstandet ist und zwar in Schließrichtung des Griffhebels.

In anderen Worten ausgedrückt, wird gemäß der vorliegenden Offenbarung eine Vorrichtung zum Öffnen und Schließen bereitgestellt, mit einer Tür oder Klappe eines Gerätegehäuses und einem Handgriff der an einer ein erstes Scharnier bildenden Griffachse schwenkbar an einem Klappen- oder Tür-Gehäuse angebracht ist und ein Hebelgelenk, das jeweils an einer ein zweites Scharnier bildenden Hebelgelenkachse mit dem Handgriff und an einer ein drittes Scharnier bildenden Hebelgelenkachse mit einer am Klappen- oder Tür-Gehäuse relativ dazu längsgeführten Schieberkulisse schwenkbar verbunden ist, wobei die das dritte Scharnier bildende Hebelgelenkachse und die das erste Scharnier bildende Griffachse auf einer imaginären Linie liegen; wobei sich die das zweite Scharnier ausbildende Hebelgelenkachse bei geschlossener Position des Handgriffs auf einer Seite der imaginären Linie in einer Öffnungsrichtung des Handgriffs befindet und bei der geöffneter Position des Handgriffs auf der anderen Seite der imaginären Linie befindet, wobei die Schieberkulisse eine Eingriffs-Längsnut aufweist, die sich in Schieberkulissen-Schließrichtung öffnet, derart, dass bei einem Verschieben der Schieberkulisse in Schließposition infolge eines Verschwenkens des Handgriffs in Schließrichtung ein auf Seiten des Gerätegehäuses angeordneter Bolzen in die Eingriffs-Längsnut eingeleitet und so die schwenkbar am Geräte-Gehäuse gelagerte Klappe oder Tür gegen das Gerätegehäuse anzieht.

D.h., die erfindungsgemäße Vorrichtung zum Öffnen und Schließen weist den Griff/ Handgriff auf, der mittels einer Griffachse schwenkbar an einem Gehäuse/Klappe/Tür beispielsweise einer medizinischen Pumpe angebracht ist. Des Weiteren weist die erfindungsgemäße Vorrichtung zum Öffnen und Schließen das Hebelgelenk (Pleuel) auf, das jeweils an einem Ende an der zweiten Hebelgelenkachse mit dem Griff und an der dritten Hebelgelenkachse mit der Schieberkulisse schwenkbar verbunden ist. Die dritte Hebelgelenkachse und die erste Griffachse liegen auf einer imaginären (Verbindungs-)Linie. Die zweite Hebelgelenkachse befindet sich bei (vollständig) geschlossener Position des Griffs vor der imaginären Linie (in Betätigungsrichtung des Griffhebels gesehen) und bei geöffneter Position des Griffs, bei der die Vorrichtung eine Gegenlagerrolle freigibt, dahinter (ebenfalls in Betätigungsrichtung des Griffhebels gesehen).

Im Konkreten ist der Griffhebel der erfindungsgemäßen Vorrichtung zum Öffnen und Schließen angepasst und geeignet, rotatorisch bzw. drehbar über eine erste Griffachse/ Griffrotationsachse an der Tür/Klappe oder dergleichen gelagert zu sein, welche beispielsweise an einem Pumpengehäuse anscharniert ist. Dabei ist der Griff/Griffhebel über die Griffachse verlängert (unter Ausbildung des Aktionsabschnitts) und dort mittels der drehbaren/rotatorischen zweiten Hebelgelenkachse mit dem Hebelgelenk somit drehbar/rotatorisch verbunden. Das Hebelgelenk, das an dem einen Ende mittels der zweiten Hebelgelenkachse mit dem Griff verbunden ist, weist an seinem anderen Ende die dritte Hebelgelenkachse auf und ist damit rotatorisch/drehbar mit der Schieberkulisse verbunden.

Die gerade imaginäre Linie zwischen der dritten Hebelgelenkachse und der ersten Griffachse bildet eine imaginäre Achse. Die zweite Hebelgelenkachse befindet sich dabei, wenn sich der Griff in einer Schließstellung befindet, also sich in seiner Schließendposition befindet, unterhalb dieser imaginären Achse. Unterhalb der imaginären Achse bedeutet dabei, dass er sich auf der Seite befindet, in die der Griff zum Öffnen gezogen bzw. aufgeklappt wird. In wieder anderen Worten ist unterhalb der imaginären Achse die Richtung der Gehäuse-/Klappenaußenseite.

Durch die vorgehende beschriebene Anordnung rotiert die zweite Hebelgelenkachse (Scharnier) auf einem kreisrunden Umfang um die erste Griffachse (Scharnier) und gleichzeitig auch um die dritte Hebelgelenkachse (Scharnier), da diese zusammen mit der Schieberkulisse längsgeführt ist. Da der Griff einen verlängerten Hebel an der ersten Griffachse darstellt wird damit ein Kniehebeleffekt erreicht. Durch diese Kniehebelanordnung sowie infolge der vorstehend definierten Raumgeometrie bewegt sich die Schieberkulisse beim Öffnen aus der Schließendposition des Griffhebels zuerst (geringfügig) in Schließrichtung bevor sie sich dann in Öffnungsrichtung bewegt. Dieser Effekt wird dadurch erreicht, dass sich die erste Hebelgelenkachse zuerst durch den Totpunkt (alle drei Achsen/Scharniere liegen auf einer Linie) bewegen muss.

In einer weiteren bevorzugten Ausführungsform ist der Griff/Griffhebel mittels einer Feder in Schließrichtung vorgespannt ist. Vorzugsweise ist die Feder an ihrem einen Ende gegen das Gehäuse/Klappe/Tür abgestützt, windet sich dann um die erste Griffachse und ist schließlich an ihrem anderen Ende an dem Griff abgestützt, so dass eine Vorspannung der Feder den Griff in Richtung hin zu seiner Endschließposition zwingt.

In einer weiteren bevorzugten Ausführungsform ist das Hebelgelenk (Pleuel) abgewinkelt, vorzugsweise mit einem Winkel zwischen 90° und 180° und besonders bevorzugt mit einem Winkel zwischen 120° und 145°. In anderen Worten ist das Hebelgelenk ein Winkelelement, vergleichbar zu einem Bumerang. Durch diese Form, erhält das Hebelgelenk zusätzlich die Funktion eines öffnungswinkelbegrenzenden Anschlags für den Griff, der sich in maximaler Offenstellung in die Kehle des abgewinkelten Hebelgelenks einfügt. Auf diese Weise kann der durch das Hebelgelenk gebildete Anschlag den maximalen Öffnungswinkel des Griffs auf kleiner gleich 90°, besonders bevorzugt 85° bezüglich der Schließendposition begrenzen.

In einer weiteren bevorzugten Ausführungsform wird die dritte Hebelgelenkachse (Scharnier) in einer Führungsnut des Gehäuses/Klappe beispielsweise einer Pumpe geführt, vorzugsweise mittels einer Rolle an der dritten Hebelgelenkachse. In anderen Worten befindet sich in dem Gehäuse der Klappe/ Tür oder ähnlichem eine Führungsnut bzw. ein Langloch, durch die die dritte Hebelgelenkachse ragt und gehalten wird. An einem Ende ist die dritte Hebelgelenkachse an dem Hebelgelenk angebracht bzw. mit diesem verbunden und an dem anderen Ende mit der Schieberkulisse, wobei dazwischen ein Abstand liegt, der als Führung/Führungsstab in der Führungsnut geführt wird. In diesem Abstand kann die Rolle oder dergleichen angebracht sein, um die Reibung zu verringern. Die Führungsnut ist dabei parallel zur Schieberkulisse und vorzugsweise parallel Gehäuseaußenseite/Klappenaußenfläche angebracht, d.h. sie öffnet sich in jene Richtung, in die der Griff geklappt wird. Die Führungsnut definiert somit die Bewegungs-/Schieberichtung des Hebelgelenks, wobei die Schieberkulisse so gelagert ist, dass deren Bewegungsrichtung in Kulissenlängsrichtung parallel zur Führungsnut ist.

In einer weiteren bevorzugten Ausführungsform wird die Schieberkulisse an der ersten Griffachse abgestützt, vorzugsweise an einer Rolle an der ersten Griffachse. In anderen Worten bietet die Schieberkulisse eine Auflagekante/Führungskante/Anschlag zum Führen an der ersten Griffachse. Die erste Griffachse, um die sich der Griff drehen kann und mittels der der Griff an dem Gehäuse/Klappe angebracht ist, ist an einem Ende, das auf der entgegengesetzten Seite der Schieberkulisse liegt in einen ersten Lagerdom an dem Gehäuse/Klappe eingebracht bzw. steckt in diesem. An dem anderen Ende der ersten Griffachse ist diese durch einen weiteren zweiten Lagerdom geführt, jedoch ist dieser im Gegensatz zu dem ersten Lagerdom ein Durchgangsloch, wodurch die erste Griffachse durch das Gehäuse/Klappe ragt und an diesem Ende einen Führungsvorsprung/Führungsanschlag für die Schieberkulisse bildet. Vorzugsweise ist an diesem Ende eine Rolle oder dergleichen angebracht, um die Reibung zwischen der ersten Griffachse und der Schieberkulisse zu verringern und ein reibungsarmes Gleiten zu ermöglichen. Gleichzeitig kann eine Rolle an dieser Stelle die Funktion haben, die erste Griffachse in ihrer Axialrichtung in dem Gehäuse zu fixieren.

In einer bevorzugten Ausführungsform nimmt die Schieberkulisse eine Gegenlagerrolle in geschlossenem Zustand in Eingriff und gibt Sie in einem offenen Zustand frei. In anderen Worten ausgedrückt, ist die Funktion der Vorrichtung zum Öffnen und Schließen einer Klappe oder dergleichen zu öffnen und sicher zu verschließen. Dies wird dadurch erreicht, dass die Schieberkulisse an der Klappe eine Gegenlagerrolle an dem Gehäuse eines Geräts/Pumpe oder dergleichen in Eingriff nimmt. Die Schieberkulisse bewegt sich in Bewegungsrichtung, die parallel zur Führungsnut und der Anschlagskante der Schieberkulisse für die erste Griffachse ist. In einer Ausführungsform kann das Hebelgelenk durch eine Feder oder dergleichen ersetzt werden, um ein besseres haptisches Feedback zu bekommen und einen höheren Gegendruck vor Erreichen des Totpunktes zu schaffen. Nach Durchlaufen des Totpunktes wie vorstehend definiert, unterstütz die Feder die Öffnung des Griffs und wirkt einer Zustellkraft entgegen.

In einer Ausführungsform umfasst das Verfahren zum Schließen und Öffnen einer Vorrichtung, einen Griff der mittels einer ersten Griffachse schwenkbar an einem Gehäuse/Klappe angebracht ist und ein Hebelgelenk das jeweils an einer zweiten Hebelgelenkachse mit dem Griff und an einer dritten Hebelgelenkachse mit einer Schieberkulisse schwenkbar verbunden ist. Die Schieberkulisse bewegt sich während des Öffnens zuerst in Schließrichtung und erst nach Durchqueren der ersten Hebelgelenkachse durch einen Totpunkt zwischen der zweiten Hebelgelenkachse und der ersten Griffachse in Öffnungsrichtung.

### Kurzbeschreibung der Figuren

Die Erfindung wird unter Bezugnahme der Zeichnungen, in welcher eine Ausführungsform beispielhaft dargestellt ist, näher beschrieben.
Fig. 1 zeigt eine perspektivische Ansicht der Verbindung von Schieberkulisse, Gelenkhebel und Handgriff der Vorrichtung zum Öffnen und Schließen.
Fig. 2 zeigt eine perspektivische Ansicht einzelner Komponenten der Vorrichtung zum Öffnen und Schließen in einem Gehäuse angeordnet.
Fig. 3 zeigt eine perspektivische Ansicht des Gehäuses der Vorrichtung zum Öffnen und Schließen ohne Komponenten.
Fig. 4 zeigt eine Draufsicht von Komponenten der Vorrichtung zum Öffnen und Schließen in geschlossenem Zustand.
Fig. 5 zeigt eine Draufsicht von Komponenten der Vorrichtung zum Öffnen und Schließen in der Totpunktstellung.
Fig. 6 zeigt eine Draufsicht von Komponenten der Vorrichtung zum Öffnen und Schließen in geöffnetem Zustand.

### Figurenbeschreibung

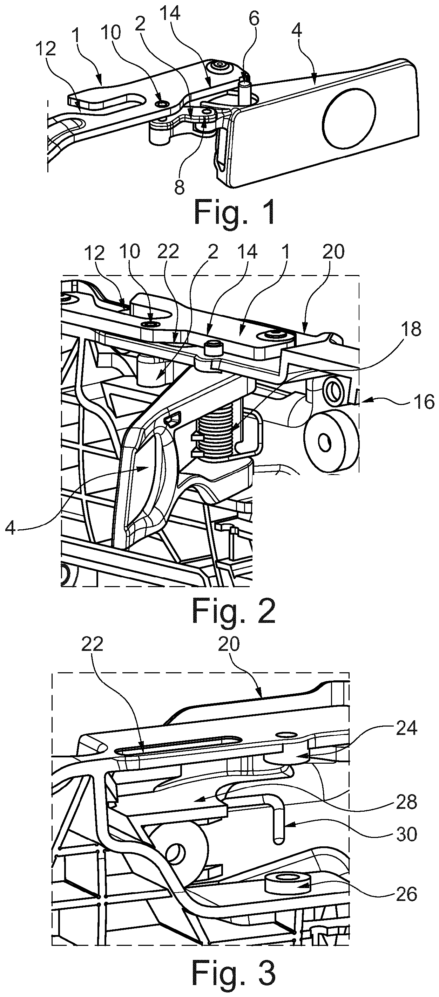

In Figur 1 ist eine perspektivische Ansicht der Verbindung von einer Schieberkulisse 1, einem Gelenkhebel 2 und einem Griff/Handgriff 4 der Vorrichtung zum Öffnen und Schließen dargestellt. Der Handgriff 4 ist drehbar auf einer (ersten) Griffachse/Klappengriffachse 6 gelagert und an seinem einen Ende, das dem anderen Griffbereich bezüglich der Griffachse 6 gegenüberliegt, über eine (zweite) Hebelgelenkachse 8 mit einem Ende des Gelenkhebels 2 drehbar verbunden. Der Gelenkhebel 2 ist wiederum an seinem anderen Ende mittels einer (dritten) Hebelgelenkachse 10 drehbar mit der Schieberkulisse 1 verbunden.

Der Griff 4 ist an seiner Vorderseite, also der Fläche des Griffs 4, die dem Bediener des Griffs 4 zugewandt ist, also einer Außenseite einer Vorrichtung/Gerätes oder dergleichen, im Wesentlichen flach ausgebildet. An seinem Griffbereich ist er ergonomisch ausgebildet, das bedeutet, auf der Rückseite sind abgerundete Ecken und Kanten für eine verbesserte, ergonomische Aufnahme der Finger. Der Griff 4 ist drehbar durch die (erste) Griffachse 6 gelagert. Der Griff 4 ist über die (erste) Griff-/Drehachse 6 hinaus ausgebildet. In anderen Worten bildet der Griff 4 auf der einen Seite der Griffachse 6 einen langen Hebel zum Ziehen und Schieben aus und auf der anderen Seite einen kürzeren Hebel zum schwenkbaren Betätigen der ersten Hebelgelenkachse 8 und somit zum Betätigen des Hebelgelenks 2 an sich, was zu einer Hebelwirkung führt.

Das Hebelgelenk 2 ist an einem Ende schwenkbar über die zweite Hebelgelenkachse 8 mit dem Griff 4 verbunden und auf der anderen Seite schwenkbar über die dritte Hebelgelenkachse 10 mit der Schieberkulisse 1 verbunden. Das Hebelgelenk 2 ist dabei auf einer Seite, die beim Öffnen des Griffs 4 mit diesem in Eingriff/Kontakt kommt kehlfömig abgewinkelt und auf der anderen Seite abgerundet. Der abgewinkelte Bereich weist vorzugsweise einen Winkel zwischen 90° und 180° und besonders bevorzugt einen Winkel zwischen 120 und 145° auf.

Die Schieberkulisse 1 ist flach ausgestaltet, vorzugsweise durch Bandstahl. Die Schieberkulisse 1 weist wenigsten eine Eingriffs-Längsnut 12 auf, die sich in Schieberichtung der Schieberkulisse 1, insbesondere in Schließ-Schieberichtung öffnet, derart, dass bei einem Verschieben der beispielsweise an einer Klappe gelagerten Schieberkulisse 1 in Schließendposition ein auf Seiten eines Gerätegehäuses (z.B. Gehäuse einer medizinischen Pumpe) angeordneter Bolzen 32 in die Eingriffs-Längsnut 12 eingeleitet und so die Schwenkbar am Gehäuse gelagerte Klappe gegen das Gehäuse anzieht. Des Weiteren weist die Schieberkulisse 1 eine Anschlagsfläche/ Anschlagskante/ Führungskante/ Führungsfläche 14 auf, die in Richtung Klappenvorderseite weist. Diese Anschlagsfläche oder Anschlagskante/Führungskante 14 ist in Kontakt mit der (ersten) Griffachse 4 und wird von dieser an einem Verdrehen um die dritte Hebelgelenkachse 10 gehindert. Die (erste) Griffachse 4 weist vorzugsweise eine Rolle auf, um Reibung zwischen sich und der Schieberkulisse 1 zu minimieren.

In Figur 2 wird eine perspektivische Ansicht einzelner Komponenten der Vorrichtung zum Öffnen und Schließen gezeigt, die in dem Gehäuse 16 der Klappe/Tür angeordnet sind. In anderen Worten werden die Schieberkulisse 1, das Hebelgelenk 2, der Griff 4 und eine Feder 18 in montiertem/eingebauten Zustand gezeigt.

Die Schieberkulisse 1 liegt demnach auf ihrer flachen Seite auf dem Gehäuse 16 der Klappe auf, genauer gesagt auf dem oberen Rand der Klappe oder dergleichen. Während die Schieberkulisse 1 auf einer Seite die Anschlagskante 14 ausbildet, an der das Ende der (ersten) Griffachse 4 gleitend in Kontakt ist, wird auf der gegenüberliegenden Seite der Schieberkulisse 1 diese von einer Schieberkulissenführung 20 des Klappen-Gehäuses 16 gehalten bzw. geführt. An der oberen Seite des Klappen-Gehäuses 16, also bei der perspektivischen Ansicht der Figuren 1 oder 3 oben, ist eine Führungsnut 22 angebracht. Die Führungsnut 22 ist als Langloch ausgebildet, in der ein oberer Abschnitt einer dritten Hebelgelenkachse 10 geführt wird, die zwischen der Schieberkulisse 1 und dem Hebelgelenk 2 als drittes Scharnier angeordnet ist. Das Langloch 22 ist parallel zu der Längsachse der Schieberkulisse 1 angeordnet. In anderen Worten liegt die Schieberkulisse 1 auf dem Klappen-Gehäuse 16 auf und ist mit dem Gelenkhebel 2 über die (dritte) Hebelgelenkachse 10 verbunden, der wiederum mit dem Griff 4 über die (zweite) Hebelgelenkachse 6 verbunden ist.

In Figur 3 wird das Gehäuse 16 der Klappe allein, ohne weitere Komponenten dargestellt. Der Griff 4 ist durch die Feder 18 in Schließrichtung, also in Richtung hin zu der Außenseite der Klappe federvorgespannt und wird indirekt durch die Feder 18 an dem Klappen-Gehäuse 16 und direkt durch einen Durchgangslagerdom für den Klappengriff 24 an der oberen Seite der Draufsicht der Figur 2, also der Seite, die der Schieberkulisse 1 zugewandt ist, und einen Auflagelagerdom für den Klappengriff 26 an der unteren Seite gelagert. In anderen Worten sitzt die Griffachse 6 auf/in dem Klappen-Gehäuse 16 an/in dem unteren Lagerdom 26 auf während sie durch den oberen Durchgangslagerdom 24 hindurchgeführt wird und durch diese hindurch auf der anderen Seite des Klappen-Gehäuses 16 herausragt. Das Hebelgelenk 2 befindet sich in einem Bauraum 28 innerhalb des Klappengehäuses 16, in dem es rotieren kann.

Die Feder 18 wird an einem Enden/Federbein in einer Federaufnahme 30 des Klappen-Gehäuses 16 gehalten. Die Feder 18 ist in ihrem Mittenabschnitt mehrmals um die Griffachse 6 gewunden und an ihrem anderen Ende/Federbein an dem Griff 4 so befestigt/gehalten, dass die Feder 18 den Griff 4 in Schließrichtung, also der Position die der Griff 4 bei geschlossenem Zustand einnimmt, vorgespannt.

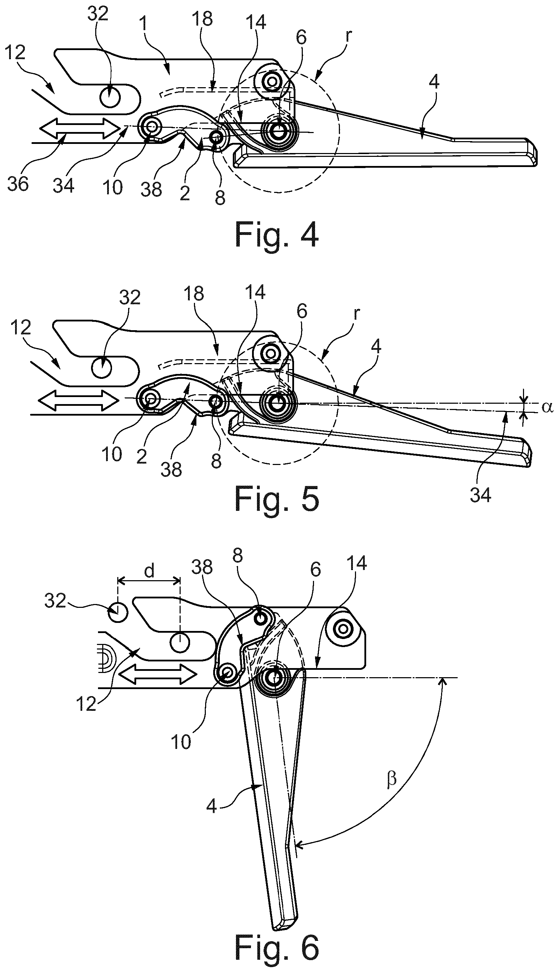

In Figur 4 ist eine Draufsicht von Komponenten der Vorrichtung zum Öffnen und Schließen in geschlossenem Zustand dargestellt. In anderen Worten zeigt die Figur 4 den Zustand, wenn sich der Griff in seiner Schließendposition befindet. In dieser Position befindet sich der Gerätegehäuse-seitige Bolzen 32 in der dafür vorgesehenen und angepassten Längsnut 12, sodass die Klappe fest gegen das Gerätegehäuse gezogen ist. Zwischen der dritten Hebelgelenkachse 10 und der ersten Griffachse 6 wird eine imaginäre Linie gezogen, sozusagen eine imaginäre Achse 34. In der Figur 4, die die Draufsicht zeigt, ist in der geschlossenen Endstellung unterhalb, also in Richtung hin zur Außenseite der Klappe, die zweite Hebelgelenkachse 8 angeordnet. Bei einer Betätigung des Griffs 4, also beim Öffnen des Griffs 4, bewegt sich die Schieberkulisse 1 in Richtung des Pfeils 36 zunächst von der ersten Griffachse weg und dann zu der ersten Griffachse hin. Die Bewegungsrichtung 36 gibt dabei die Achse an, auf der sich die Schieberkulisse 1 bewegen kann. Da die Schieberkulisse 1 durch die dritte Hebelgelenkachse 10 bewegt wird, die durch die Führungsnut 22 geführt wird, ist die Bewegung der Schieberkulisse 1 parallel zu der Führungsnut 22.

Wird der Griff 4 weiter betätigt, also gezogen, und rotiert somit im Uhrzeigersinn um die (erste) Griffachse 6 wird die (zweite) Hebelgelenkachse 8 auf einem Radius r um die Griffachse 6 im Uhrzeigersinn geführt und erreicht die Totpunktstellung wie in Figur 5 gezeigt. Die Totpunktstellung ist die Stellung, in der sich die zweite Hebelgelenkachse 8 auf der imaginären Achse 34 zwischen der (ersten) Griffachse 6 und der dritten Hebelgelenkachse 10 befindet. Gleichzeitig rotiert die zweite Hebelgelenkachse 8 auch auf einem Radius um die dritte Hebelgelenkachse 10. Dadurch, dass die dritte Hebelgelenkachse 10 in der Führungsnut 22 in Bewegungsrichtung beweglich ist wird der Abstand zwischen der dritten Hebelgelenkachse 10 und der (ersten) Griffachse 6 größer, was darin resultiert, dass sich die Schieberkulisse 1 in ihrer Axialrichtung von der (ersten) Griffachse 6 in Bewegungsrichtung 36 in Richtung zum Bolzen 32 hin entfernt. Vorzugsweise bewegt sich die Schieberkulisse 1, durch die Drehung des Griffs 4 und das dadurch verursachte Positionieren der zweiten Hebelgelenkachse 6 auf die imaginäre Achse 34, um ca. 0,3mm. Der Griff wird dabei vorzugsweise um den Winkel *α* von ca. 6° zu der Achse der Bewegungsrichtung 36 in Richtung zur Klappenvorderseite bewegt. Durch eine weitere Betätigung des Griffs 4 verlässt die zweite Hebelgelenkachse 8 den Totpunkt wieder und wandert weiter im Uhrzeigersinn auf dem Radius r um die (erste) Griffachse 6. Dadurch ändert sich die Richtung, in der die Schieberkulisse 1 bewegt wird, und die Schieberkulisse 1 entfernt sich vom Bolzen 32. Der Abstand zwischen der dritten Hebelgelenkachse 10 und der (ersten) Griffachse 4 verringert sich.

In Figur 6 wird die Vorrichtung zum Öffnen und Schließen in ihrer vollständigen OffenPosition gezeigt. Dabei befindet sich der Griff 4 in einer Position in der er einen Winkel β ca. 85° bezüglich der Längsachse der Schieberkulisse 1 zurückgelegt hat. Die Schieberkulisse 1 legt dabei eine Strecke d von ca. 15 mm zurück, wodurch der Bolzen 32 aus der Führungsnut 12 geführt wird. Das Hebelgelenk 2 bildet einen Anschlag 38 aus, der durch den Winkel an der Seite des Hebelgelenks 2 definiert wird. Der Anschlag 38 begrenzt die relative Drehung des Griffs 4 in Offenrichtung, vorzugsweise auf einen Winkel von ca. 85°. In anderen Worten ausgedrückt wirken der Griff 4 und das Hebelgelenk 2 nach dem Kniehebelprinzip zusammen, wobei der Griff 4 durch den Anschlag 38 auf den relativen Öffnungswinkel begrenzt wird.

### Bezugszeichenliste

- 1: Schieberkulisse
- 2: Hebelgelenk
- 4: Griff/ Klappengriff
- 6: Klappengriffachse (erstes Scharnier)
- 8: Hebelgelenkachse (zweites Scharnier)
- 10: Hebelgelenkachse (drittes Scharnier)
- 12: Gelenklagerrollenführung/Führungsnut
- 14: Anschlagsfläche/ Führungsfläche
- 16: Klappen-Gehäuse
- 18: Feder
- 20: Schieberkulissenführung
- 22: Führungsnut
- 24: Durchgangslagerdom für Klappengriff
- 26: Aufsetzlagerdom für Klappengriff
- 28: Bauraum für Hebelgelenk
- 30: Federaufnahme
- 32: Gegenlagerrolle/Bolzen
- 34: Imaginäre Achse
- 36: Bewegungsrichtung Schieberkulisse
- 38: Anschlag
- *α*: Winkel *α*
- *β*: Winkel β
- d: Abstand d
- r: Radius r

## Patentansprüche

1. Pumpenghäuse umfassend eine Klappe oder Tür und eine Vorrichtung zum Öffnen und Schließen der Klappe oder Tür, die Vorrichtung aufweisend: einen Handgriff (4) der an einer ein erstes Scharnier bildenden Griffachse (6) schwenkbar an einem Klappen- oder Tür-Gehäuse (16) angebracht ist und ein Hebelgelenk (2), das jeweils an einer ein zweites Scharnier bildenden Hebelgelenkachse (8) mit dem Handgriff (4) und an einer ein drittes Scharnier bildenden Hebelgelenkachse (10) mit einer am Klappen- oder Tür-Gehäuse (16) relativ dazu längsgeführten Schieberkulisse (1) schwenkbar verbunden ist, wobei die das dritte Scharnier bildende Hebelgelenkachse (10) und die das erste Scharnier bildende Griffachse (6) auf einer imaginären Linie (34) liegen; wobei sich die das zweite Scharnier ausbildende Hebelgelenkachse (8) bei geschlossener Position des Handgriffs (4) auf einer Seite der imaginären Linie (34) in einer Öffnungsrichtung des Handgriffs (4) befindet und bei der geöffneter Position des Handgriffs (4) auf der anderen Seite der imaginären Linie (34) befindet, wobei die Schieberkulisse (1) eine Eingriffs-Längsnut (12) aufweist, die sich in Schieberkulissen-Schließrichtung öffnet, derart, dass bei einem Verschieben der Schieberkulisse (1) in Schließposition infolge eines Verschwenkens des Handgriffs (4) in Schließrichtung ein auf Seiten des Pumpengehäuses angeordneter Bolzen (32) in die Eingriffs-Längsnut (12) eingleitet und so die schwenkbar am Pumpengehäuse gelagerte Klappe oder Tür gegen das Pumpengehäuse anzieht, wobei die Schieberkulisse (1) eine Anschlagsfläche/-kante oder Führungsfläche-/kante (14) aufweist, die in Richtung Klappenvorderseite weist, wobei die Anschlagsfläche/-kante oder Führungsfläche-Z kante (14) in Kontakt mit einer Griffachse (4) ist und von dieser an einem Verdrehen um die dritte Hebelgelenkachse (10) gehindert wird.

2. Pumpengehäuse nach Anspruch 1, wobei der Handgriff (4) mittels einer Feder (18) in Schließrichtung vorgespannt ist.

3. Pumpengehäuse nach Anspruch 1 oder 2, wobei das Hebelgelenk (2) abgewinkelt ist, vorzugsweise mit einem Winkel zwischen 90° und 180° und besonders bevorzugt mit einem Winkel zwischen 120° und 145°.

4. Pumpengehäuse nach einem der vorgehenden Ansprüche, wobei das Hebelgelenk (2) einen für den Handgriff (4) öffnungswinkelbegrenzenden Anschlag (38) aufweist oder bildet, wobei der Anschlag (38) den Öffnungswinkel des Handgriffs (4) zur Bewegungsrichtung vorzugsweise auf ca. 85° begrenzt.

5. Pumpengehäuse nach einem der vorgehenden Ansprüche, wobei die das dritte Scharnier bildende Hebelgelenkachse (10) gleichzeitig für eine Führung in einer Führungsnut (22) des Klappen-Gehäuses (16) ausgebildet ist.

6. Pumpengehäuse nach einem der vorgehenden Ansprüche, wobei sich die Schieberkulisse (1) an der Griffachse (6) abstützt, vorzugsweise an einer Rolle an dem Ende der Griffachse (6).

7. Pumpengehäuse nach Anspruch 5, wobei die Schieberkulisse (1) dafür ausgebildet ist, um längs der Führungsnut (22) geführt zu werden, wobei die Führungsnut (22) vorzugsweise parallel zu einer Gehäusevorderseite angeordnet ist.

8. Pumpengehäuse nach einem der vorgehenden Ansprüche, wobei die Schieberkulisse (1) den Bolzen (32) in geschlossenem Zustand in Eingriff nimmt und diesen im offenem Zustand freigibt.

9. Verfahren zum Schließen und Öffnen eines Pumpengehäuses nach einem der Ansprüche 1 bis 8, wobei sich während des Verschwenkens des Handgriffs (4) in Öffnungsrichtung die Schieberkulisse (1) aus der Schließposition in die Offenposition zuerst in Schließrichtung bewegt und nach Durchqueren der ersten Hebelgelenkachse (8) durch einen Totpunkt zwischen der das dritte Scharnier ausbildenden Hebelgelenkachse (10) und der das erste Scharnier ausbildenden Griffachse (4) weiter in Öffnungsrichtung bewegt.

## Claims

1. A pump housing comprising a flap or door and a device for opening and closing the flap or door, the device comprising: a handle (4) that is pivotally attached to a flap or door housing (16) on a handle axis (6) forming a first hinge, and a lever joint (2), which is respectively connected pivotably to the handle (4) on a lever joint axis (8) forming a second hinge, and, on a lever joint axis (10) forming a third hinge, to a slide (1) which is guided longitudinally on the flap or door housing (16) relative to the latter, the lever joint axis (10) forming the third hinge and the handle axis (6) forming the first hinge lying on an imaginary line (34); wherein the lever joint axis (8) forming the second hinge, in the closed position of the handle (4), is located on one side of the imaginary line (34) in an opening direction of the handle (4) and, in the open position of the handle (4), is located on the other side of the imaginary line (34), the slide (1) having an engagement longitudinal groove (12) which opens in the closing direction of the slide such that, when the slide (1) is shifted in the closed position due to pivoting of the handle (4) in the closing direction, a bolt (32) arranged on the side of the pump housing slides into the engagement longitudinal groove (12) and thus tightens the flap or door pivotably mounted on the pump housing to the pump housing, wherein the slide (1) has a contact surface/ edge or guide surface/edge (14) which points in the direction of the front side of the flap, the contact surface/ edge or guide surface/ edge (14) being in contact with a handle axis (4) and being prevented by the latter from turning about the third lever joint axis (10).

2. The pump housing according to claim 1, wherein the handle (4) is prestressed in the closing direction by means of a spring (18).

3. The pump housing according to claim 1 or 2, wherein the lever joint (2) is angled, preferably at an angle between 90° and 180° and particularly preferably at an angle between 120° and 145°.

4. The pump housing according to any of the preceding claims, wherein the lever joint (2) has or forms a stop (38) limiting the opening angle of the handle (4), wherein the stop (38) preferably limits the opening angle of the handle (4) to preferably about 85° with respect to the direction of movement.

5. The pump housing according to any of the preceding claims, wherein the lever joint axis (10) forming the third hinge is at the same time configured for guidance in a guide groove (22) of the flap housing (16).

6. The pump housing according to any of the preceding claims, wherein the slide (1) is supported on the handle axle (6), preferably on a roller at the end of the handle axle (6).

7. The pump housing according to claim 5, wherein the slide (1) is configured to be guided along the guide groove (22), wherein the guide groove (22) is preferably arranged parallel to a front side of the housing.

8. The pump housing according to any of the preceding claims, wherein the slide (1) engages the bolt (32) in the closed state and releases it in the open state.

9. A method for closing and opening a pump housing according to any of claims 1 to 8, wherein during pivoting of the handle (4) in the opening direction the slide (1) first moves from a closing position to an open position in the closing direction and, after passing through the first lever joint axis (8) through a dead center between the lever joint axis (10) forming the third hinge and the handle axis (4) forming the first hinge, further moves in the opening direction.

## Revendications

1. Boîtier de pompe comprenant un clapet ou une porte et un dispositif pour ouvrir et fermer le clapet ou la porte, le dispositif présentant : une poignée (4) qui est placée de manière pivotante sur un boîtier de clapet ou de porte (16) au niveau d'un axe de poignée (6) formant une première charnière et une articulation de levier (2) qui est reliée de manière pivotante à la poignée (4) respectivement au niveau d'un axe d'articulation de levier (8) formant une deuxième charnière et au niveau d'un axe d'articulation de levier (10) formant une troisième charnière à une coulisse de glissière (1) guidée longitudinalement par rapport au boîtier de clapet ou de porte (16), dans lequel l'axe d'articulation de levier (10) formant la troisième charnière et l'axe de poignée (6) formant la première charnière se trouvent sur une ligne imaginaire (34) ;
dans lequel l'axe d'articulation de levier (8) formant la deuxième charnière se situe, en position fermée de la poignée (4), d'un côté de la ligne imaginaire (34) dans une direction d'ouverture de la poignée (4) et se situe, en position ouverte de la poignée (4), de l'autre côté de la ligne imaginaire (34), dans lequel la coulisse de glissière (1) présente une rainure longitudinale d'engagement (12) qui s'ouvre dans la direction de fermeture de coulisse de glissière de sorte que, lors d'un déplacement de la coulisse de glissière (1) en position de fermeture suite à un pivotement de la poignée (4) dans la direction de fermeture, un boulon (32) disposé sur des côtés du boîtier de pompe glisse dans la rainure longitudinale d'engagement (12) et serre ainsi sur le boîtier de pompe le clapet ou la porte monté de manière pivotante sur le boîtier de pompe, dans lequel la coulisse de glissière (1) présente une surface/arête de butée ou une surface/arête de guidage (14) qui est orientée en direction du côté avant de clapet, dans lequel la surface/arête de butée ou la surface/arête de guidage (14) est en contact avec un axe de poignée (4) et est empêchée par celui-ci de tourner autour du troisième axe d'articulation de levier (10).

2. Boîtier de pompe selon la revendication 1, dans lequel la poignée (4) est précontrainte dans la direction de fermeture au moyen d'un ressort (18).

3. Boîtier de pompe selon la revendication 1 ou 2, dans lequel l'articulation de levier (2) est coudée, de préférence selon un angle entre 90° et 180° et le plus préférentiellement selon un angle entre 120° et 145°.

4. Boîtier de pompe selon l'une quelconque des revendications précédentes, dans lequel l'articulation de levier (2) présente ou forme une butée (38) limitant l'angle d'ouverture pour la poignée (4), dans lequel la butée (38) limite l'angle d'ouverture de la poignée (4) par rapport à la direction de déplacement, de préférence à env. 85°.

5. Boîtier de pompe selon l'une quelconque des revendications précédentes, dans lequel l'axe d'articulation de levier (10) formant la troisième charnière est simultanément conçu pour un guidage dans une rainure de guidage (22) du boîtier de clapet (16).

6. Boîtier de pompe selon l'une quelconque des revendications précédentes, dans lequel la coulisse de glissière (1) s'appuie sur l'axe de poignée (6), de préférence sur un rouleau à l'extrémité de l'axe de poignée (6).

7. Boîtier de pompe selon la revendication 5, dans lequel la coulisse de glissière (1) est conçue pour être guidée le long de la rainure de guidage (22), dans lequel la rainure de guidage (22) est de préférence agencée de manière parallèle à un côté avant de boîtier.

8. Boîtier de pompe selon l'une quelconque des revendications précédentes, dans lequel la coulisse de glissière (1) engage le boulon (32) à l'état fermé et libère celui-ci à l'état ouvert.

9. Procédé de fermeture et d'ouverture d'un boîtier de pompe selon l'une quelconque des revendications 1 à 8, dans lequel, pendant le pivotement de la poignée (4) dans la direction d'ouverture, la coulisse de glissière (1) se déplace d'abord dans la direction de fermeture depuis la position de fermeture vers la position d'ouverture et continue à se déplacer dans la direction d'ouverture après avoir traversé le premier axe d'articulation de levier (8) par un point mort entre l'axe d'articulation de levier (10) formant la troisième charnière et l'axe de poignée (4) formant la première charnière.
